# EUROPEAN PATENT APPLICATION

(11) **EP 4 814 011 A1**
(43) Date of publication of application: **30.09.2026**
(21) Application number: 24893500.9
(22) Date of filing: 21.11.2024
(51) Int. Cl.: C07K 16/24, C12N 15/13, C12N 15/64, A61K 39/395, A61P 37/02, A61P 35/00, A61P 29/00

(54) **ANTIGEN BINDING PROTEIN TARGETING TSLP AND USE**

(30) Priority: 22.11.2023 CN 202311569843
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: YU, Haijia, Shanghai 201203 (CN); ZHU, Xiangyang, Shanghai 201203 (CN); ZHAN, Yifan, Shanghai 201203 (CN); WANG, Yanting, Shanghai 201203 (CN); WEI, Xiaoyue, Shanghai 201203 (CN); CHENG, Yong, Shanghai 201203 (CN)
(74) Representative: Weickmann & Weickmann PartmbB
(86) International application number: PCT/CN2024/133383
(87) International publication number: WO 2025/108344

(57) **Abstract**

The present application relates to an isolated antigen-binding protein targeting TSLP. The present application further provides a preparation method and use of the isolated antigen-binding protein.

## Description

### TECHNICAL FIELD

The present application relates to the field of biological medicines, and specifically relates to an antigen-binding protein targeting TSLP and a use thereof.

### BACKGROUND ART

Thymic stromal lymphopoietin (TSLP) is an interleukin-7 (IL-7)-like cytokine. Mature hTSLP is composed of 131 amino acid residues and has a typical four-stranded α-helical bundle structure. TSLP is mainly produced in epithelial cells, smooth muscle cells, keratinocytes, stromal cells, fibroblasts, mast cells (MC), monocyte-macrophages, granulocytes and dendritic cells (DC). TSLP, as an important allergic factor secreted by epithelial cells, induces the body's Th2 response by interacting with locally infiltrating DC cells and T cells. In addition, TSLP can also transform the body's inflammatory response from Th1 to Th2 by inhibiting the Th1 pathway, playing an important role in coordination and equilibrium in the inflammatory response.

A TSLP receptor complex is a heterodimer composed of a TSLP receptor (TSLPR) and an IL-7 receptor α (IL-7Rα), both of which are highly expressed in myeloid dendritic cells. After binding to TSLPR on the cell membrane, TSLP binds to IL-7Rα to form a stable TSLP-TSLPR-IL7Rα receptor complex. The intracellular segment of the TSLPR receptor in the complex recruits and activates JAK2, which works together with JAK1 recruited by IL7Rα to activate the downstream signaling molecule TSLP. After TSLP binding to the surface receptor of a myeloid dendritic cell, the dendritic cell secretes IL-8 and eotaxin-2 to recruit neutrophil and eosinophil, and secretes TARC and MDC to recruit a Th2 cell. In addition, a TSLP-activated DC cell induces a CD4+T cell to differentiate into a Th2 cell. The Th2 cell can produce IL4, IL-5, IL-13 and TNF. These cytokines promote the production of IgE, eosinophil and mucus to initiate an allergic reaction and trigger diseases such as asthma and atopic dermatitis.

Currently, there are few studies on TSLP-related diseases, and there is an urgent need to an antibody targeting TSLP.

### SUMMARY

The present application provides an isolated antigen-binding protein targeting TSLP, which has the following advantages: (1) capability of blocking the binding of TSLP to a TSLPR receptor on a cell surface, (2) capability of binding to human and animal TSLP proteins, (3) good thermal stability, and (4) capability of blocking the production of TARC.

The present application further provides a nucleic acid molecule encoding the isolated antigen-binding protein, an expression vector, a host cell, and a method for preparing the isolated antigen-binding protein. The isolated antigen-binding protein of the present application can be used to prevent, alleviate and/or treat diseases and/or disorders.

In one aspect, the present application provides an isolated antigen-binding protein, including HCDR3, and the HCDR3 includes an amino acid sequence as set forth in SEQ ID NO: 3.

In some embodiments, the isolated antigen-binding protein includes HCDR2, and the HCDR2 includes an amino acid sequence as set forth in SEQ ID NO: 2.

In some embodiments, the isolated antigen-binding protein includes HCDR1, and the HCDR1 includes an amino acid sequence as set forth in SEQ ID NO: 1.

In some embodiments, the isolated antigen-binding protein includes H-FR1, a C terminus of the H-FR1 is directly or indirectly connected to an N terminus of the HCDR1, and the H-FR1 includes an amino acid sequence optionally selected from SEQ ID NO: 7, SEQ ID NO: 8, and SEQ ID NO: 9.

In some embodiments, the isolated antigen-binding protein includes H-FR2, the H-FR2 is located between the HCDR1 and the HCDR2, and the H-FR2 includes an amino acid sequence optionally selected from SEQ ID NO: 10, SEQ ID NO: 11, SEQ ID NO: 12, and SEQ ID NO: 13.

In some embodiments, the isolated antigen-binding protein includes H-FR3, the H-FR3 is located between the HCDR2 and the HCDR3, and the H-FR3 includes an amino acid sequence optionally selected from SEQ ID NO: 14, SEQ ID NO: 15, SEQ ID NO: 16, SEQ ID NO: 17, and SEQ ID NO: 18.

In some embodiments, the isolated antigen-binding protein includes H-FR4, an N terminus of the H-FR4 is connected to a C terminus of the HCDR3, and the H-FR4 includes an amino acid sequence optionally selected from SEQ ID NO: 19 and SEQ ID NO: 20.

In some embodiments, the isolated antigen-binding protein includes VH, and the VH includes an amino acid sequence optionally selected from SEQ ID NO: 31, SEQ ID NO: 32, SEQ ID NO: 33, SEQ ID NO: 34, and SEQ ID NO: 35.

In some embodiments, the isolated antigen-binding protein includes LCDR3, and the LCDR3 includes an amino acid sequence as set forth in SEQ ID NO: 6.

In some embodiments, the isolated antigen-binding protein includes LCDR2, and the LCDR2 includes an amino acid sequence as set forth in SEQ ID NO: 5(GAR).

In some embodiments, the isolated antigen-binding protein includes LCDR1, and the LCDR1 includes an amino acid sequence as set forth in SEQ ID NO: 4.

In some embodiments, the isolated antigen-binding protein includes L-FR1, a C terminus of the L-FR1 is directly or indirectly connected to an N terminus of the LCDR1, and the L-FR1 includes an amino acid sequence optionally selected from SEQ ID NO: 21 and SEQ ID NO: 22.

In some embodiments, the isolated antigen-binding protein includes L-FR2, the L-FR2 is located between the LCDR1 and the LCDR2, and the L-FR2 includes an amino acid sequence optionally selected from SEQ ID NO: 23, SEQ ID NO: 24, and SEQ ID NO: 25.

In some embodiments, the isolated antigen-binding protein includes L-FR3, the L-FR3 is located between the LCDR2 and the LCDR3, and the L-FR3 includes an amino acid sequence optionally selected from SEQ ID NO: 26, SEQ ID NO: 27, SEQ ID NO: 28, and SEQ ID NO: 29.

In some embodiments, the isolated antigen-binding protein includes L-FR4, an N terminus of the L-FR4 is connected to a C terminus of the LCDR3, and the L-FR4 includes an amino acid sequence that may be as set forth in SEQ ID NO: 30.

In some embodiments, the isolated antigen-binding protein includes VL, and the VL includes an amino acid sequence optionally selected from SEQ ID NO: 36, SEQ ID NO: 37, SEQ ID NO: 38, and SEQ ID NO: 39.

In some embodiments, the isolated antigen-binding protein includes any one group of VH and VL selected from the group consisting of:
1) VH: SEQ ID NO: 31, VL: SEQ ID NO: 36;
2) VH: SEQ ID NO: 32, VL: SEQ ID NO: 37;
3) VH: SEQ ID NO: 32, VL: SEQ ID NO: 38;
4) VH: SEQ ID NO: 32, VL: SEQ ID NO: 39;
5) VH: SEQ ID NO: 33, VL: SEQ ID NO: 37;
6) VH: SEQ ID NO: 33, VL: SEQ ID NO: 38;
7) VH: SEQ ID NO: 33, VL: SEQ ID NO: 39;
8) VH: SEQ ID NO: 34, VL: SEQ ID NO: 37;
9) VH: SEQ ID NO: 34, VL: SEQ ID NO: 38;
10) VH: SEQ ID NO: 34, VL: SEQ ID NO: 39;
11) VH: SEQ ID NO: 35, VL: SEQ ID NO: 37;
12) VH: SEQ ID NO: 35, VL: SEQ ID NO: 38; and
13) VH: SEQ ID NO: 35, VL: SEQ ID NO: 39.

In some embodiments, the isolated antigen-binding protein includes a constant region of the antibody heavy chain.

In some embodiments, the constant region of the antibody heavy chain is derived from a human IgG heavy chain constant region.

In some embodiments, the constant region of the antibody heavy chain is derived from a human IgG1 heavy chain constant region.

In some embodiments, the isolated antigen-binding protein includes a constant region of the antibody light chain.

In some embodiments, the constant region of the antibody light chain is derived from a human Igκ constant region.

In some embodiments, the isolated antigen-binding protein includes an antibody or an antigen-binding fragment thereof.

In some embodiments, the antigen-binding fragment includes Fab, Fab', Fv fragment, F(ab')2, F(ab)2, scFv, di-scFv, and/or dAb.

In some embodiments, the antibody is selected from one or more of the group consisting of: monoclonal antibodies, chimeric antibodies, humanized antibodies, fully human antibodies.

In another aspect, the present application provides a chimeric antigen receptor, including a targeting moiety, and the targeting moiety includes the isolated antigen-binding protein of the present application.

In another aspect, the present application provides a polypeptide molecule, including the isolated antigen-binding protein or the chimeric antigen receptor.

In some embodiments, the polypeptide molecule includes a fusion protein.

In another aspect, the present application provides an immunoconjugate, including the isolated antigen-binding protein.

In another aspect, the present application provides one or more isolated nucleic acid molecules, encoding the isolated antigen-binding protein, the chimeric antigen receptor, or the polypeptide molecule.

In another aspect, the present application provides a vector, including the nucleic acid molecule.

In another aspect, the present application provides a cell, including the isolated antigen-binding protein, the chimeric antigen receptor, the polypeptide molecule, the immunoconjugate, the nucleic acid molecule, or the vector.

In another aspect, the present application provides a pharmaceutical composition, including the isolated antigen-binding protein, the chimeric antigen receptor, the polypeptide molecule, the immunoconjugate, the nucleic acid molecule, the vector, and/or the cell, and optionally a pharmaceutically acceptable carrier.

In another aspect, the present application provides a method for preparing the isolated antigen-binding protein, including: culturing the cell under a condition that allows expression of the antigen-binding protein.

In another aspect, the present application provides a use of the isolated antigen-binding protein, the chimeric antigen receptor, the polypeptide molecule, the immunoconjugate, the nucleic acid molecule, the vector, the cell, and/or the pharmaceutical composition in the manufacture of a medicament for preventing, alleviating, and/or treating diseases and/or disorders.

In some embodiments, the diseases and/or disorders include TSLP related diseases.

In some embodiments, the diseases and/or disorders include inflammatory diseases or tumors.

In some embodiments, the diseases and/or disorders include TSLP related inflammatory diseases or tumors.

In another aspect, the present application provides a method for detecting TSLP in a sample, including application of the isolated antigen-binding protein, the chimeric antigen receptor, the polypeptide molecule, the immunoconjugate, the nucleic acid molecule, the vector, the cell, and/or the pharmaceutical composition.

In another aspect, the present application provides a reagent or kit for detecting TSLP in a sample, including the isolated antigen-binding protein, the chimeric antigen receptor, the polypeptide molecule, the immunoconjugate, the nucleic acid molecule, the vector, the cell, and/or the pharmaceutical composition.

In another aspect, the present application provides a use of the isolated antigen-binding protein, the chimeric antigen receptor, the polypeptide molecule, the immunoconjugate, the nucleic acid molecule, the vector, the cell, and/or the pharmaceutical composition in preparation of a kit for detecting the presence and/or content of TSLP in a sample.

Those skilled in the art can easily discern other aspects and advantages of the present application from the following detailed description. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. A brief description of the drawings is as follows:
FIG. 1 shows the curves of the activity and EC50 values of the 900792 and related humanized proteins of the present application binding to TSLP on a cell membrane surface.
FIG. 2 shows the curves and related IC50 values of the 900792 and related humanized proteins of the present application blocking TSLP from binding to receptors on a cell surface.
FIG. 3 shows the curves and related IC50 values of the 900792 and related humanized proteins of the present application in inhibiting the functional activity of TSLP binding to receptors on a cell surface.
FIG. 4 shows the ELISA test results of the 900792 humanized proteins of the present application inhibiting the TARC activity.
FIG. 5 shows the ELISA test results of the 900792 humanized proteins of the present application inhibiting the TARC activity.

### DETAILED DESCRIPTION

The embodiments of the invention of the present application will be described below with specific examples. Those skilled in the art can easily understand other advantages and effects of the invention of the present application from the disclosure of the specification.

### Definition of Terms

In the present application, the term "TSLP", also called "Thymic stromal lymphopoietin", is an interleukin-7 (IL-7)-like cytokine. Mature hTSLP is composed of 131 amino acid residues and has a typical four-stranded α-helical bundle structure. The term "TSLP" covers "full-length", unprocessed TSLP, and any form of TSLP produced by cell processing. In the present application, the term "TSLP" includes full-length, wild-type TSLP as well as mutants, fragments, variants, isoforms and homologues thereof.

In the present application, the term "isolated" typically refers to artificial collection from a natural state. If an "isolated" substance or component occurs in nature, it may be that the natural environment in which the substance or component is has changed, or that the substance has been isolated from the natural environment, or both. For example, a certain unisolated polynucleotide or polypeptide naturally exists in a living animal, and the same polynucleotide or polypeptide with high purity isolated from the natural state is called "isolated". The term "isolated" does not exclude the presence of artificial or synthetic substances, nor other impure substances that do not affect the activity of the substances.

In the present application, the term "isolated antigen-binding protein" typically refers to a protein which is collected from a natural state by an artificial means and has an antigen-binding ability. The "isolated antigen-binding protein" may include an antigen-binding moiety, and optionally, a framework or frame moiety that allows the antigen-binding moiety to use a conformation that promotes the antigen-binding moiety to bind to an antigen. The antigen-binding protein may include, for example, antibody-derived protein framework regions (FRs) or alternative protein framework regions or artificial framework regions with grafted CDRs or CDR derivatives. Such frameworks include, but are not limited to, antibody-derived framework regions including mutations introduced, for example, to stabilize the three-dimensional structure of an antigen-binding protein, as well as fully synthetic framework regions including, for example, biocompatible polymers. Examples of antigen-binding proteins include, but are not limited to: human antibodies and humanized antibodies; chimeric antibodies; recombinant antibodies; single-chain antibodies; bifunctional antibodies; trifunctional antibodies; tetrafunctional antibodies; Fab, Fab', Fv fragment, F(ab')₂, F(ab)₂, scFv, di-scFv, dAb, IgD antibodies; IgE antibodies; IgM antibodies; IgG1 antibodies; IgG2 antibodies; IgG3 antibodies; or IgG4 antibodies and fragments thereof.

In the present application, the term "CDR", also known as a "complementarity determining region", typically refers to a region in an antibody variable domain, the sequence of which is highly variable and/or forms a structure defining loop. Typically, an antibody includes six CDRs: three (HCDR1, HCDR2, and HCDR3) in VH, and three (LCDR1, LCDR2, and LCDR3) in VL. In some embodiments, a naturally occurring camel antibody consisting only of heavy chains can function normally and stably even in the absence of light chains. An antibody CDR may be determined by a variety of encoding systems, e.g., CCG, Kabat, Chothia, IMGT, and Kabat/Chothia considered comprehensively. These encoding systems are known in the art. For example, the amino acid sequence numbering of the antigen-binding proteins may be divided according to an IMGT numbering system. For example, the CDRs of the antigen-binding proteins may be divided according to a Kabat numbering system.

In the present application, the term "FR" typically refers to a more highly conserved moiety of an antibody variable domain, called a framework region. Typically, the variable domains of the natural heavy chain and light chain each includes four FR regions, i.e., four (H-FR1, H-FR2, H-FR3, and H-FR4) in VH, and four (L-FR1, L-FR2, L-FR3, and L-FR4) in VL.

In the present application, the terms "variable domain" and "variable region" may be used interchangeably, and typically refer to a moiety of a heavy chain and/or a light chain of an antibody. The variable domains of a heavy chain and a light chain may be referred to as "V_{H}" and "V_{L}" (or "VH" and "VL"), respectively. These domains are typically the most variable moieties of an antibody (relative to other antibodies of the same type), and include antigen-binding sites.

In the present application, the term "chimeric antigen receptor" (CAR) typically refers to a recombinant polypeptide including at least an extracellular domain that specifically binds to an antigen or target, a transmembrane region, and an intracellular domain. For example, a hinge region is included between the extracellular domain and the transmembrane region. For example, the chimeric antigen receptor may include a signal peptide. The binding of an extracellular domain of CAR to a target antigen on the surface of a target cell leads to CAR clustering and transmits the activation stimulus to a CAR-containing cell. The CAR redirects the specificity of an immune effector cell, and triggers proliferation, cytokine production, as well as phagocytosis and/or production of molecules capable of mediating cell death of a target antigen-expressing cell in a major histocompatibility (MHC)-independent manner. For example, the extracellular structure may include the aforementioned antigen-binding protein. For example, the extracellular structure may specifically bind to TSLP.

In the present application, the terms "polypeptide molecule", "polypeptide", and "peptide" may be used interchangeably and typically refer to polymers of amino acid residues. The term "fusion protein" typically refers to a polypeptide consisting of at least two moieties covalently linked together. Each moiety may be a polypeptide having different properties. The properties may be biological properties, such as in vitro or in vivo activity. The properties may also be simple chemical or physical properties, e.g., binding to target molecules, and catalysis of reactions. The two moieties may be linked directly by a single peptide bond or a peptide linker.

In the present application, the term "nucleic acid molecule" typically refers to a nucleotide, deoxyribonucleotide, or ribonucleotide in an isolated form and of any length, or an analogue isolated from its natural environment or artificially synthesized.

In the present application, the term "vector" typically refers to a nucleic acid carrying tool into which polynucleotide that encodes a certain protein may be inserted and the protein is expressed. A vector may be transformed, transduced, or transfected into a host cell to express a genetic material element it carries within the host cell. For example, vectors may include plasmids; phagemids; Cosmids; artificial chromosomes such as yeast artificial chromosomes (YAC), bacterial artificial chromosomes (BAC), or P1 derived artificial chromosomes (PAC); phages such as λ phage or M13 phage; animal viruses; and the like. The types of animal viruses used as vectors include retroviruses (including lentiviruses), adenoviruses, adeno-associated viruses, herpes viruses (e.g., herpes simplex virus), poxviruses, baculoviruses, papillomaviruses, and papovaviruses (e.g., SV40). A vector may include a variety of elements that control expression, including promoter sequences, transcription initiation sequences, enhancer sequences, selection elements, and reporter genes. In addition, the vector may also include a replication initiation site. The vector may also include components that facilitate to enter cells, such as viral particles, liposomes, or protein coats, which are not limited thereto.

In the present application, the term "cell" generally refers to a single cell, cell line, or cell culture that may be, or has been a recipient of a subject plasmid or vector, which includes the nucleic acid molecule of the present application or the vector of the present application. The cell may include the progeny of a single cell. Due to natural, accidental or intentional mutations, the progeny may not necessarily be identical to the original mother cell (in the morphology of the total DNA complement or in genome). The cell may include a cell transfected in vitro by the vector of the present application. The cell may be a bacterial cell (e.g., E. coli), a yeast cell, or any other eukaryotic cell, e.g., a COS cell, Chinese hamster ovary (CHO) cell, CHO-K1 cell, LNCAP cell, HeLa cell, HEK293 cell, COS-1 cell, or NS0 cell. In some embodiments, the cell is a mammalian cell. In some embodiments, the mammalian cell is an HEK293 cell.

In the present application, the term "immunoconjugate" typically refers to a conjugate formed by conjugating other agents (e.g., chemotherapeutic agents, radioactive elements, cytostatic agents, and cytotoxic agents) to the antibody or an antigen binding fragment thereof (e.g., covalently linked by a linker molecule), and the conjugate may deliver the agents to a target cell (e.g., a tumor cell) by the antibody or the antigen binding fragment thereof specifically binding to an antigen on the target cell.

In the present application, the term "pharmaceutical composition" typically refers to a composition used for preventing/treating diseases or disorders. The pharmaceutical composition may include the isolated antigen-binding protein of the present application, the nucleic acid molecule of the present application, the vector of the present application, and/or the cell of the present application, and optionally a pharmaceutically acceptable adjuvant. In addition, the pharmaceutical composition may further include suitable preparations of one or more (pharmaceutically effective) carriers, stabilizers, excipients, diluents, solubilizers, surfactants, emulsifiers and/or preservatives. The acceptable components of the composition at the doses and concentrations used are preferably non-toxic to the recipients. The pharmaceutical composition of the present application includes, but is not limited to, a liquid, frozen or freeze-dried composition.

In the present application, the term "pharmaceutically acceptable carrier" typically includes a pharmaceutically acceptable carrier, excipient or stabilizer that is non-toxic at the doses and concentrations used to a cell or mammal exposed thereto. Physiologically acceptable carriers may include, for example, buffering agents, antioxidants, low molecular weight (about 10 residues or less) polypeptides, proteins, hydrophilic polymers, amino acids, monosaccharides, disaccharides and other carbohydrates, chelating agents, sugar alcohols, salt forming counter ions (e.g., sodium), and/or non-ionic surfactants.

In the present application, the term "subject" typically refers to a human or a non-human animal, including, but is not limited to a cat, dog, horse, pig, cow, sheep, rabbit, mouse, rat, or monkey.

In the present application, it should also be understood that a protein, polypeptide, and/or amino acid sequence involved including at least the following scope: variants or homologues having the functions same as or similar to the protein or the polypeptide.

In the present application, the variants may be proteins or polypeptides obtained by substitution, deletion or addition of one or more amino acids in the amino acid sequence of the protein and/or the polypeptide (e.g., specifically binding to an antibody or a fragment thereof of a TSLP protein). For example, the functional variants may include proteins or polypeptides with amino acid alterations through substitution, deletion, and/or insertion of at least 1, e.g., 1-30, 1-20, 1-10, 1, 2, 3, 4 or 5 amino acids. The functional variants may substantially maintain the biological properties of the protein or the polypeptide prior to the alterations (e.g., substitution, deletion, or addition). For example, the functional variants may maintain at least 60%, 70%, 80%, 90%, or 100% of the biological activity (e.g., antigen-binding ability) of the protein or the polypeptide prior to the alterations. For example, the substitution may be conservative substitution.

In the present application, the homologues may be proteins or polypeptides having at least about 85% (e.g., about 85%, about 90%, about 91%, about 92%, about 93%, about 94%, about 95%, about 96%, about 97%, about 98%, about 99% or higher) sequence homology with the amino acid sequence of the protein and/or the polypeptide (e.g., specifically binding to the antibody or the fragment thereof of a TSLP protein).

In the present application, the homology typically refers to the similarity, likeness or association between two or more sequences. The "percentage of sequence homology" may be calculated by the following method: comparing two sequences to be aligned in a comparison window, the number of sites where same nucleic acid bases (e.g., A, T, C, G, and I) or same amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are present in the two sequences are determined to obtain the number of matching sites, the number of matching sites is divided by the total number of sites (i.e., the window size) in the comparison window, and the result is multiplied by 100 to obtain the percentage of sequence homology. The alignment conducted for determining the percentage of sequence homology may be implemented by multiple methods known in the art.

In the present application, the term "include" typically refers to the meaning of include, comprise, contain, or encompass. In some cases, the term "include" also means "be" and "consist of...".

In the present application, the term "about" typically refers to a change in a range of 0.5-10% greater or less than a specified value, e.g., a change in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% greater or less than the specified value.

### Detailed Description of the Invention

### Isolated antigen-binding protein

CDR of an antibody, also known as a complementarity-determining region, is moiety of a variable region. An amino acid residue in this region may come into contact with an antigen or antigenic epitope. An antibody CDR may be determined by a variety of encoding systems, e.g., CCG, Kabat, Chothia, IMGT, and Kabat/Chothia considered comprehensively. These encoding systems are known in the art, and specific reference may be made to, for example, http://www.bioinf.org.uk/abs/index.html#kabatnum. Those skilled in the art may use different encoding systems to determine the CDR regions according to the sequences and structures of antibodies. Using different encoding systems, the CDR regions may differ. In the present application, the CDR encompasses CDR sequences divided according to any CDR division method. The CDR also encompasses variants thereof including the amino acid sequences of the CDR subjected to substitution, deletion and/or addition of one or more amino acids, e.g., substitution, deletion and/or insertion of 1-30, 1-20, 1-10, 1, 2, 3, 4, 5, 6, 7, 8 or 9 amino acids. The CDR also encompasses the homologues thereof which may have the amino acid sequences having at least about 85% (e.g., about 85%, about 90%, about 91%, about 92%, about 93%, about 94%, about 95%, about 96%, about 97%, about 98%, about 99% or higher) sequence homology with the amino acid sequence of the CDR. In some embodiments, the CDR is determined by an IMGT numbering scheme.

In one aspect, the present application provides an isolated antigen-binding protein, including HCDR3, and the HCDR3 includes an amino acid sequence as set forth in SEQ ID NO: 3.

In the present application, the isolated antigen-binding protein may further include HCDR2, and the HCDR2 may include an amino acid sequence as set forth in SEQ ID NO: 2.

In the present application, the isolated antigen-binding protein may further include HCDR1, and the HCDR1 may include an amino acid sequence as set forth in SEQ ID NO: 1.

In the present application, the isolated antigen-binding protein may include HCDR3, HCDR2 and HCDR1. For example, in the isolated antigen-binding protein, the HCDR3 includes an amino acid sequence as set forth in SEQ ID NO: 3, the HCDR2 includes an amino acid sequence as set forth in SEQ ID NO: 2, and the HCDR1 includes an amino acid sequence as set forth in SEQ ID NO: 1.

In the present application, the isolated antigen-binding protein may include LCDR3, and the LCDR3 may include an amino acid sequence as set forth in SEQ ID NO: 6.

In the present application, the isolated antigen-binding protein may further include LCDR2, and the LCDR2 may include an amino acid sequence as set forth in SEQ ID NO: 5(GAR).

In the present application, the isolated antigen-binding protein may further include LCDR1, and the LCDR1 may include an amino acid sequence as set forth in SEQ ID NO: 4.

In the present application, the isolated antigen-binding protein may include LCDR3, LCDR2 and LCDR1. For example, in the isolated antigen-binding protein, the LCDR3 includes an amino acid sequence as set forth in SEQ ID NO: 6, the LCDR2 includes an amino acid sequence as set forth in SEQ ID NO: 5, and the LCDR1 includes an amino acid sequence as set forth in SEQ ID NO: 4.

In the present application, the isolated antigen-binding protein may include HCDR3, HCDR2, HCDR1, LCDR3, LCDR2, and LCDR1. For example, in the isolated antigen-binding protein of the present application, the HCDR3 includes an amino acid sequence as set forth in SEQ ID NO: 3, the HCDR2 includes an amino acid sequence as set forth in SEQ ID NO: 2, the HCDR1 includes an amino acid sequence as set forth in SEQ ID NO: 1, the LCDR3 includes an amino acid sequence as set forth in SEQ ID NO: 6, the LCDR2 includes an amino acid sequence as set forth in SEQ ID NO: 5 (GAR), and the LCDR1 includes an amino acid sequence as set forth in SEQ ID NO: 4.

In the present application, the isolated antigen-binding protein may include H-FR1, a C terminus of the H-FR1 may be directly or indirectly connected to an N terminus of the HCDR1, and the H-FR1 may include an amino acid sequence as set forth in SEQ ID NO: 7, SEQ ID NO: 8, or SEQ ID NO: 9.

In the present application, the isolated antigen-binding protein may include H-FR2, the H-FR2 may be located between the HCDR1 and the HCDR2, and the H-FR2 may include an amino acid sequence as set forth in SEQ ID NO: 10, SEQ ID NO: 11, SEQ ID NO: 12, or SEQ ID NO: 13.

In the present application, the isolated antigen-binding protein may include H-FR3, the H-FR3 may be located between the HCDR2 and the HCDR3, and the H-FR3 may include an amino acid sequence as set forth in SEQ ID NO: 14, SEQ ID NO: 15, SEQ ID NO: 16, SEQ ID NO: 17, or SEQ ID NO: 18.

In the present application, the isolated antigen-binding protein may include H-FR4, an N terminus of the H-FR4 may be connected to a C terminus of the HCDR3, and the H-FR4 may include an amino acid sequence as set forth in SEQ ID NO: 19 or SEQ ID NO: 20.

In the present application, the antigen-binding protein may include H-FR1, H-FR2, H-FR3, and H-FR4.

For example, in the isolated antigen-binding protein, the H-FR1, H-FR2, H-FR3, and H-FR4 may include amino acid sequences as set forth in H-FR1: SEQ ID NO: 7, H-FR2: SEQ ID NO: 10, H-FR3: SEQ ID NO: 14, and H-FR4: SEQ ID NO: 19 sequentially and respectively.

For example, in the isolated antigen-binding protein, the H-FR1, H-FR2, H-FR3, and H-FR4 may include amino acid sequences as set forth in H-FR1: SEQ ID NO: 8, H-FR2: SEQ ID NO: 11, H-FR3: SEQ ID NO: 15, and H-FR4: SEQ ID NO: 20 sequentially and respectively.

For example, in the isolated antigen-binding protein, the H-FR1, H-FR2, H-FR3, and H-FR4 may include amino acid sequences as set forth in H-FR1: SEQ ID NO: 9, H-FR2: SEQ ID NO: 12, H-FR3: SEQ ID NO: 16, and H-FR4: SEQ ID NO: 20 sequentially and respectively.

For example, in the isolated antigen-binding protein, the H-FR1, H-FR2, H-FR3, and H-FR4 may include amino acid sequences as set forth in H-FR1: SEQ ID NO: 8, H-FR2: SEQ ID NO: 13, H-FR3: SEQ ID NO: 17, and H-FR4: SEQ ID NO: 20 sequentially and respectively.

For example, in the isolated antigen-binding protein, the H-FR1, H-FR2, H-FR3, and H-FR4 may include amino acid sequences as set forth in H-FR1: SEQ ID NO: 9, H-FR2: SEQ ID NO: 12, H-FR3: SEQ ID NO: 18, and H-FR4: SEQ ID NO: 20 sequentially and respectively.

In the present application, the isolated antigen-binding protein may include L-FR1, a C terminus of the L-FR1 may be directly or indirectly connected to an N terminus of the LCDR1, and the L-FR1 may include an amino acid sequence as set forth in SEQ ID NO: 21 or SEQ ID NO: 22.

In the present application, the isolated antigen-binding protein may include L-FR2, the L-FR2 may be located between the LCDR1 and the LCDR2, and the L-FR2 may include an amino acid sequence as set forth in SEQ ID NO: 23, SEQ ID NO: 24, or SEQ ID NO: 25.

In the present application, the isolated antigen-binding protein may include L-FR3, the L-FR3 may be located between the LCDR2 and the LCDR3, and the L-FR3 may include an amino acid sequence as set forth in SEQ ID NO: 26, SEQ ID NO: 27, SEQ ID NO: 28, or SEQ ID NO: 29.

In the present application, the isolated antigen-binding protein may include L-FR4, an N terminus of the L-FR4 may be connected to a C terminus of the LCDR3, and the L-FR4 may include an amino acid sequence as set forth in SEQ ID NO: 30.

In the present application, the antigen-binding protein may include L-FR1, L-FR2, L-FR3, and L-FR4.

For example, in the isolated antigen-binding protein, the L-FR1, L-FR2, L-FR3, and L-FR4 may include amino acid sequences as set forth in L-FR1: SEQ ID NO: 21, L-FR2: SEQ ID NO: 23, L-FR3: SEQ ID NO: 26, and L-FR4: SEQ ID NO: 30 sequentially and respectively.

For example, in the isolated antigen-binding protein, the L-FR1, L-FR2, L-FR3, and L-FR4 may include amino acid sequences as set forth in L-FR1: SEQ ID NO: 22, L-FR2: SEQ ID NO: 24, L-FR3: SEQ ID NO: 27, and L-FR4: SEQ ID NO: 30 sequentially and respectively.

For example, in the isolated antigen-binding protein, the L-FR1, L-FR2, L-FR3, and L-FR4 may include amino acid sequences as set forth in L-FR1: SEQ ID NO: 22, L-FR2: SEQ ID NO: 24, L-FR3: SEQ ID NO: 28, and L-FR4: SEQ ID NO: 30 sequentially and respectively.

For example, in the isolated antigen-binding protein, the L-FR1, L-FR2, L-FR3, and L-FR4 may include amino acid sequences as set forth in L-FR1: SEQ ID NO: 22, L-FR2: SEQ ID NO: 25, L-FR3: SEQ ID NO: 29, and L-FR4: SEQ ID NO: 30 sequentially and respectively.

In the present application, the isolated antigen-binding protein may include VH, and the VH may include an amino acid sequence as set forth in SEQ ID NO: 31, SEQ ID NO: 32, SEQ ID NO: 33, SEQ ID NO: 34, or SEQ ID NO: 35.

In the present application, the isolated antigen-binding protein may include VL, and the VL may include an amino acid sequence as set forth in SEQ ID NO: 36, SEQ ID NO: 37, SEQ ID NO: 38, or SEQ ID NO: 39.

In the present application, the isolated antigen-binding protein may include the VH and the VL.

For example, the VH may include an amino acid sequence as set forth in SEQ ID NO: 31, and the VL may include an amino acid sequence as set forth in SEQ ID NO: 36.

For example, the VH may include an amino acid sequence as set forth in SEQ ID NO: 32, and the VL may include an amino acid sequence as set forth in SEQ ID NO: 37.

For example, the VH may include an amino acid sequence as set forth in SEQ ID NO: 32, and the VL may include an amino acid sequence as set forth in SEQ ID NO: 38.

For example, the VH may include an amino acid sequence as set forth in SEQ ID NO: 32, and the VL may include an amino acid sequence as set forth in SEQ ID NO: 39.

For example, the VH may include an amino acid sequence as set forth in SEQ ID NO: 33, and the VL may include an amino acid sequence as set forth in SEQ ID NO: 37.

For example, the VH may include an amino acid sequence as set forth in SEQ ID NO: 33, and the VL may include an amino acid sequence as set forth in SEQ ID NO: 38.

For example, the VH may include an amino acid sequence as set forth in SEQ ID NO: 33, and the VL may include an amino acid sequence as set forth in SEQ ID NO: 39.

For example, the VH may include an amino acid sequence as set forth in SEQ ID NO: 34, and the VL may include an amino acid sequence as set forth in SEQ ID NO: 37.

For example, the VH may include an amino acid sequence as set forth in SEQ ID NO: 34, and the VL may include an amino acid sequence as set forth in SEQ ID NO: 38.

For example, the VH may include an amino acid sequence as set forth in SEQ ID NO: 34, and the VL may include an amino acid sequence as set forth in SEQ ID NO: 39.

For example, the VH may include an amino acid sequence as set forth in SEQ ID NO: 35, and the VL may include an amino acid sequence as set forth in SEQ ID NO: 37.

For example, the VH may include an amino acid sequence as set forth in SEQ ID NO: 35, and the VL may include an amino acid sequence as set forth in SEQ ID NO: 38.

For example, the VH may include an amino acid sequence as set forth in SEQ ID NO: 35, and the VL may include an amino acid sequence as set forth in SEQ ID NO: 39.

In the present application, the isolated antigen-binding protein may include at least one CDR in the VH of the present application. In the present application, the isolated antigen-binding protein may include at least one CDR in the VL of the present application. The CDR may be obtained by dividing according to any division method.

In the present application, the isolated antigen-binding protein may include HCDR1, HCDR2, and HCDR3 in the VH of the present application. The VH may include an amino acid sequence as set forth in any one of SEQ ID NO: 31, SEQ ID NO: 32, SEQ ID NO: 33, SEQ ID NO: 34, and SEQ ID NO: 35.

In the present application, the isolated antigen-binding protein may include LCDR1, LCDR2, and LCDR3 in the VL of the present application. The VL may include an amino acid sequence as set forth in any one of SEQ ID NO: 36, SEQ ID NO: 37, SEQ ID NO: 38, and SEQ ID NO: 39.

In the present application, the isolated antigen-binding protein may include a constant region of the antibody heavy chain. The constant region of the antibody heavy chain may be derived from a human IgG heavy chain constant region. In some embodiments, the isolated antigen-binding protein may include a constant region of the antibody heavy chain, and the constant region of the antibody heavy chain may be derived from a human IgG1 heavy chain constant region.

In the present application, the isolated antigen-binding protein may include a constant region of the antibody light chain. The constant region of the antibody light chain may be derived from a human Igκ constant region.

In the present application, the isolated antigen-binding protein may include an antibody or an antigen-binding fragment thereof.

In some embodiments, the antigen-binding fragment may include Fab, Fab', Fv fragment, F(ab')₂, F(ab)₂, scFv, di-scFv, and/or dAb.

In some embodiments, the antibody may include monoclonal antibodies, chimeric antibodies, humanized antibodies, and/or fully human antibodies.

In addition, it is to be noted that the isolated antigen-binding protein of the present application may include a heavy chain and/or light chain sequence having one or more conserved sequence modifications present in the heavy chain and/or light chain sequence. The "conserved sequence modifications" refer to amino acid modifications that do not significantly affect or change the antibody binding properties. Such conserved modifications include substitution, addition, and deletion of amino acids. The modification may be introduced into the isolated antigen-binding proteins of the present application by standard techniques known in the art, e.g., point mutations and PCR-mediated mutations. The conserved amino acid substitution refers to the substitution of amino acid residues with amino acid residues having similar side chains. The groups of amino acid residues having similar side chains are known in the art. These groups of amino acid residues include those with basic side chains (e.g., lysine, arginine, and histidine), acidic side chains (e.g., aspartic acid, and glutamic acid), uncharged polar side chain (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine) and aromatic side chains (e.g., tyrosine, phenylalanine, tryptophan, and histidine). In some embodiments, one or more amino acid residues in the CDR region of the isolated antigen-binding protein of the present application may be substituted with other amino acid residues of the same side chain group. Those skilled in the art know that some conserved sequence modifications do not cause loss of antigen binding activity.

### Chimeric antigen receptor, polypeptide molecule, nucleic acid molecule, vector, cell, immunoconjugate, and pharmaceutical composition

In another aspect, the present application provides a chimeric antigen receptor (CAR), and the chimeric antigen receptor (CAR) may include a targeting moiety that binds to a TSLP protein.

In another aspect, the present application provides a polypeptide molecule, which may include the isolated antigen-binding protein of the present application.

In some embodiments, the polypeptide molecule may include a fusion protein. In some embodiments, the polypeptide molecule may include a fusion protein.

In another aspect, the present application provides an isolated nucleic acid molecule, which may encode the isolated antigen-binding protein of the present application. For example, the nucleic acid molecule may be produced or synthesized by: (1) amplification in vitro, e.g., by polymerase chain reaction (PCR); (2) clonal recombination; (3) purification, e.g., by enzymatic digestion and gel electrophoresis fractionation; or (4) synthesis, e.g., chemical synthesis.

In another aspect, the present application provides a vector, which may include the nucleic acid molecule of the present application. In addition, the vector may further include other genes, for example, a marker gene that allows selection of the vector in an appropriate host cell and under an appropriate condition. In addition, the vector may further include an expression control element that allows correct expression of a coding region in an appropriate host. Such a control element is well known to those skilled in the art, and may be, for example, a promoter, a ribosome binding site, an enhancer, and other control elements that regulate gene transcription or mRNA translation. The vector may be transformed, transduced, or transfected into a host cell to express a genetic material element it carries within the host cell. The vector may be, for example, a plasmid, a cosmid, a virus, a phage, or other vectors commonly used in, for example, genetic engineering. For example, the vector is an expression vector. In addition, the vector may also include a component that facilitates entry into a cell, e.g., a viral particle, a liposome, or a protein coat, which are not limited thereto.

In another aspect, the present application provides a cell, which may include the nucleic acid molecule of the present application, or the vector of the present application. In some embodiments, each type of or each host cell may include one or one type of the nucleic acid molecule or the vector of the present application. In some embodiments, each type of or each host cell may include multiple (e.g., 2 or more) or multiple types (e.g., 2 or more types) of the nucleic acid molecules or the vectors of the present application. For example, the vector of the present application may be introduced into the host cell, e.g., a eukaryotic cell, such as a plant-derived cell, a fungus, or a yeast cell. In some embodiments, the cell may be a bacterial cell (e.g., E. coli), yeast cell, or any other eukaryotic cell, e.g., COS cell, Chinese hamster ovary (CHO) cell, CHO-K1 cell, LNCAP cell, HeLa cell, 293T cell, COS-1 cell, SP2/0 cell, NS0 cell, or myeloma cell. The vector of the present application may be introduced into the host cell by methods known in the art, e.g., electroporation, lipofectine transfection, and lipofectamin transfection.

In another aspect, the present application provides an immunoconjugate, which may include the isolated antigen-binding protein of the present application.

In another aspect, the present application provides a pharmaceutical composition, which may include the isolated antigen-binding protein of the present application, the polypeptide molecule of the present application, the immunoconjugate of the present application, the nucleic acid molecule of the present application, the vector of the present application, and/or the cell of the present application, and optionally a pharmaceutically acceptable carrier.

In some embodiments, the pharmaceutical composition may include suitable preparations of one or more (pharmaceutically effective) adjuvants, stabilizers, excipients, diluents, solubilizers, surfactants, emulsifiers and/or preservatives. The acceptable components of the composition at the dose and concentration used are preferably non-toxic to a recipient. The pharmaceutical composition of the present application includes, but is not limited to, a liquid, frozen or freeze-dried composition.

In some embodiments, the pharmaceutical composition may be a bispecific or multispecific molecule including the isolated antigen-binding protein of the present application. In some embodiments, in addition to the molecules that bind to TSLP, the targets bound by other molecules in the bispecific or multispecific molecules may be unrelated to TSLP.

In some embodiments, the pharmaceutical composition may also include one or more active compounds, typically those which have complementary activity and do not adversely affect one another. The type and effective dosage of such drugs may depend on for example the amount and type of an antagonist present in a preparation, as well as clinical parameters of subjects.

In some embodiments, the pharmaceutically acceptable carrier may include any and all solvents, dispersion media, coatings, isotonic agents, and absorption delay agents that are compatible with drug administration, typically safe and non-toxic.

### Preparation method

In another aspect, the present application provides a method for preparing the antigen-binding protein. The method may include: the host cell of the present application is cultured under a condition that allows expression of the antigen-binding protein. For example, methods using appropriate medium, appropriate temperature and culture time, and the like are known to those skilled in the art.

Any method suitable for producing monoclonal antibodies may be used to produce the antigen-binding protein of the present application. For example, an animal may be immunized with linked or naturally occurring TSLP or a fragment thereof. Suitable immunization methods may be used, including adjuvant, immunostimulant, and repeated booster immunizations, and one or more routes may be used. For example, a hybridoma preparation method may be used, wherein spleen cells are obtained from immunized mice, the spleen cells are fused with SP2/0 myeloma cells, and hybridoma cell lines are screened through HAT.

Any suitable form of TSLP may be used as an immunogen (antigen) to produce a non-human antibody specific to TSLP, and the biological activity of the antibody is screened. For example, an immunogen for stimulating may be full-length mature human TSLP, including a native homodimer, or a peptide containing single/multiple epitopes. The immunogen may be used alone or in combination with one or more immunogenicity enhancers known in the art.

A chimeric human antibody may be selected from any type of immunoglobulins, including IgM, IgD, IgG, IgA, and IgE. In the present application, the antibody may be an IgG antibody, and IgG1 subtype may be used. Optimization of required constant domain sequences may be achieved by screening antibodies using the biological assays described in the Examples below, to produce the desired biological activity. Likewise, either type of light chain may be used in the compounds and methods of the present application. For example, a κ chain or a variant thereof may be used in the compounds and methods of the present application.

### Method and use

In another aspect, the present application provides use of the isolated antigen-binding protein, the chimeric antigen receptor, the polypeptide molecule, the nucleic acid molecule, the vector, the cell, the immunoconjugate, and/or the pharmaceutical composition in the manufacture of a medicament for preventing and/or treating diseases and/or disorders.

In another aspect, the present application provides a method for preventing and/or treating diseases and/or disorders, and the method may include: the isolated antigen-binding protein, the chimeric antigen receptor, the polypeptide molecule, the nucleic acid molecule, the vector, the cell, the immunoconjugate, and/or the pharmaceutical composition of the present application is administered to subjects in need.

In another aspect, the isolated antigen-binding protein, the polypeptide molecule, the nucleic acid molecule, the vector, the cell, the immunoconjugate, and/or the pharmaceutical composition of the present application may be used for preventing and/or treating diseases and/or disorders.

In the present application, the diseases and/or disorders may be TSLP related diseases and/or disorders.

In the present application, the diseases and/or disorders may be inflammatory diseases or tumors.

In the present application, the diseases and/or disorders may be TSLP related inflammatory diseases or tumors.

In another aspect, the present application provides a method for detecting TSLP in a sample, including application of the isolated antigen-binding protein, the polypeptide molecule, the nucleic acid molecule, the vector, the cell, the immunoconjugate, and/or the pharmaceutical composition.

In certain situations, the method for detecting TSLP in a sample is an in vitro method. In certain situations, the method for detecting TSLP in a sample is used for non-therapeutic purposes. In certain situations, the method for detecting TSLP in a sample is not a diagnostic method.

In another aspect, the present application provides a reagent or kit for detecting TSLP in a sample, including the isolated antigen-binding protein, the polypeptide molecule, the nucleic acid molecule, the vector, the cell, the immunoconjugate, and/or the pharmaceutical composition.

In another aspect, the present application provides a use of the isolated antigen-binding protein, the polypeptide molecule, the nucleic acid molecule, the vector, the cell, the immunoconjugate, and/or the pharmaceutical composition in preparation of a kit for detecting the presence and/or content of TSLP in a sample.

Not intended to be limited by any theory, the following examples are merely intended to explain the fusion protein, the preparation method, the use, and the like of the present application, and are not intended to limit the scope of the present application.

### Examples

### Example 1 Preparation of anti-human TSLP mouse monoclonal antibodies (original antibodies)

### Preparation of hybridoma cells for producing mouse monoclonal antibodies

A hybridoma preparation technology invented by Kohler and Milstein in 1975 (Nature, 1975, 256: 495-497) was used for preparing mouse monoclonal antibodies. First, human TSLP with His tag proteins (ACRO, TSP-H52Ha) was used as the immune antigen. The Freund's Adjuvant, Complete (Sigma cat no. F5881, FCA for short) and Freund's Adjuvant, Incomplete (Sigma cat no. F5506, FICA for short) were used for immunization, and multiple BALB/c and CD1 mice were immunized subcutaneously at multiple sites. After four immunizations, serum was collected to test the titer by ELISA and the binding activity and functional activity by FACS. Finally, the optimum mouse was selected to obtain spleen cells to be fused with SP2/0 myeloma cells. Hybridoma cell lines were screened by HAT. Monoclonal hybridoma cell lines that specifically bind to human TSLP and monkey TSLP were screened out from the cell culture supernatant by FACS. Monoclonal cell lines that block TSLP-TSLPR signals and inhibit Hu-TSLP-stimulated proliferation of Baf3-overexpressing TSLPR/IL7Ra cells were further screened out. The screened monoclonal cell lines were screened for affinity (Biacore). Finally, monoclonal hybridoma cell lines for producing anti-human TSLP antibodies were obtained for sequence analysis. The screening data is listed in Table 1.

**Table 1 Data results of hybridoma screening**

| Clone | Antigen-antibody binding activity assay | | Blocking activity | Inhibitory activity | Affinity (KD) |
|---|---|---|---|---|---|
| | Human TSLP (MFI value) | Monkey TSLP (MFI value) | | | |
| 2D2 | 56460 | 6206 | 79.80% | 18.01% | 1.53E-08 |
| 2G5 | 56244 | 6393 | 95.99% | 17.91 | 1.47E-08 |
| 3C10 | 71738 | 312 | 82.94% | 19.98% | 1.19E-10 |
| 11B5 | 85284 | 267 | 93.48% | 14.44% | 7.47E-11 |
| 14A1 | 43530 | 5340 | 88.25% | 10.93% | 1.53E-08 |
| 14H4 | 42782 | 5438 | 98.57% | 10.85% | 1.55E-08 |
| 15H1 | 51830 | 5136 | 71.43% | 12.44% | 2.09E-09 |
| 23E7 | 83168 | 260 | 94.66% | 6.05% | 2.01E-10 |
| 24B6 | 74339 | 6876 | 96.06% | 4.03% | 1.11E-08 |
| 24D12 | 72234 | 6305 | 101.71% | 1.94% | 5.12E-10 |
| 28C12 | 60746 | 6487 | 90.27% | 6.33% | 1.68E-08 |
| 28H5 | 62273 | 6153 | 89.99% | 2.01% | 2.33E-08 |
| 33A6 | 43570 | 5284 | 87.90% | 2.87% | 1.67E-08 |
| 47D3 | 53426 | 830 | 98.70% | 16.58% | 1.48E-12 |
| 77E6 | 61700 | 608 | 76.56% | -0.76% | 8.09E-11 |
| 82A3 | 83692 | 224 | 95.88% | -1.16% | 2.13E-09 |
| 82A11 | 79701 | 217 | 110.95% | -0.90% | 2.10E-09 |
| 86E3 | 71768 | 6697 | 59.76% | 18.66% | 5.62E-09 |
| 90C8 | 72962 | 129 | 68.55% | -0.96% | 1.36E-09 |
| 91A11 | 78710 | 7784 | 67.45% | 18.12% | 2.15E-10 |
| 104B1 | 79680 | 7223 | 59.03% | 15.01% | 3.59E-10 |
| 110G1 | 46242 | 4087 | 38.18% | 15.91% | 1.54E-10 |
| 120E5 | 70177 | 291 | 50.10% | 17.71 % | 3.04E-10 |

### Example 2 Cloning and humanization of gene sequences of variable regions of anti-TSLP antibodies

### 2.1 Gene cloning of variable regions of antibodies in hybridoma cells

Total RNAs were extracted from the mouse hybridoma cells using QIAGEN's RNA extraction kit (#74181) according to the manufacturer's instructions. The cDNA sequences of the variable regions of the mouse antibodies expressed by the hybridoma cell lines were cloned based on the principle of TAKARA's 5'RACE technology. Briefly, gene-specific cDNAs of the variable regions of the heavy chain and light chains were synthesized using SMARTer 5'RACE Synthesis Kit (TAKARA, #634859) according to the instructions. The 5' and 3' ends of the cDNA sequences were modified by PCR primers designed to add appropriate leading sequences to the cDNAs of the variable regions of the heavy chain and light chain, respectively. Thus the resulting PCR products could be cloned onto a heavy chain vector pHB-Fc and a light chain vector pHB-Cκ expressed by existing recombinant antibodies by seamless cloning. The pHB-Fc expression vector includes the gene sequence of a human IgG1 constant region of the heavy chain, in which CH2 carries antibody ADCC effect weakened L234A and L235A (Eu numbering) mutations. The pHB-Cκ vector includes the gene sequence of a human κ constant region of the light chain. The PCR amplification products of the variable regions of the heavy chain and light chain were cloned into expression vectors using In-fusion cloning reagent (TAKARA, #639650) to obtain expression vectors of human-mouse chimeric antibodies, which were then transformed into DH5α competent cells of E. coli (Yeastern Biotech, #FYE607-80VL). Monoclonal colonies were selected for conducting Sanger sequencing, and the sequences of the variable regions of the hybridoma clone 77E6 antibodies were obtained by analysis. The sequences of the variable regions of the constructed anti-TSLP chimeric antibodies 900792 are as follows:
>900792 VH (SEQ ID NO: 31) 
QVQLQQSGAELVRPGTSVLVSCKASGYAFTNYLIEWVLQRPGQGLEWIGVINPESGDT
YYSEKFKGKATLTADKSSSTAYMQLSSLTSEDSAVYFCAKESLTGSSYDYWGQGTTLTVS
S
>900792 VL (SEQ ID NO: 36)

DIQMTQSPASLSASVGESVTITCRASENIYSNLVWYQQKQGKSPQLLVYGARNLADG
VPSRFSGSGSGTQFSLKINSLQPEDFGNYYCQHFYETPLTFGTGTKLEIK

**Table 2 CDR sequences of mouse anti-TSLP antibodies (using IMGT numbering rules)**

| Domain | | Sequence | SEQ ID NO. |
|---|---|---|---|
| VH | CDR1 | GYAFTNYL | SEQ ID NO: 1 |
| | CDR2 | INPESGDT | SEQ ID NO: 2 |
| | CDR3 | AKESLTGSSYDY | SEQ ID NO: 3 |
| VL | CDR1 | ENIYSN | SEQ ID NO: 4 |
| | CDR2 | GAR | SEQ ID NO: 5 |
| | CDR3 | QHFYETPLT | SEQ ID NO: 6 |

### 2.2 Expression of chimeric antibodies

The expression vectors obtained in 2.1 were amplified by E. coli, and a sufficient number of plasmids were prepared using an endotoxin-free plasmid extraction kit (Tiangen Biotech (Beijing) Co., Ltd., #DP117) for transient transfection and expression of chimeric antibodies. The host cells used for expression were CHO-S cells (Thermo Fisher, #R80007). The prepared two heavy chain vectors together with the light chain vectors were mixed with polyetherimide (PEI, Polysciences, Cat. No. 24765-1) separately to form liposome complexes which were transfected into the CHO-S cells, and the CHO-S cells were cultured in an incubator for 5-7 days. The supernatant of the cell medium was collected by centrifugation and purified by a Protein A affinity chromatography column to obtain human-mouse chimeric antibodies.

### 2.3 Humanization of mouse anti-human TSLP antibodies

Humanization of antigen-binding proteins was conducted by a 3D modeling method: first, 3D structural modeling was conducted on the mouse antigen-binding proteins, the optimal structural model is selected, and 5-10 optimal structural solutions were selected by a homology modeling method. A Loop region was typically modeled by the homology modeling method. If the results of CDR amino acid sequence alignment showed a 50% Identity or less, a CDR3 structural model was constructed using a de novo modeling method. 10 antibody crystal structural models with the closest sequences (having a structural resolution of 2.5 angstroms or higher) were retrieved by PDB BLAST, and compared with the automatic model to select the optimal structural model. The sequences of the variable regions of the antigen-binding proteins were then compared with available sequences in the NCBI IgBlast database. By identification and analysis, human framework regions (FR regions) suitable for constructing CDR-grafted heavy chains and light chains thereon were finally determined.

During reconstruction, the reconstruction sites were designed based on conserved amino acid residues in the FR regions of human antibodies and crucial amino acid residues in the FR regions of antibodies. Humanized mutation design was conducted on the variable regions of the heavy chains and light chains of the antigen-binding proteins 900792 of the present application respectively. The designed humanized sequences should not affect the structural stability of the antibodies, not affect the binding of the antigen-binding proteins to antigens, not introduce protein modification sites for glycosylation, phosphorylation, and the like, not introduce sites that are easily oxidized, aminated, and the like, and enhance the structural stability. The sequences of the mouse antigen-binding proteins of 900792 were analyzed, and a total of 4 humanized heavy chain sequences and 3 humanized light chain sequences were designed, which are shown as follows:
900792 heavy chain humanized sequences:
   900792-VH1 (SEQ ID NO: 32) 
QVQLQQSGAEVKKPGASVKVSCKASGYAFTNYLIEWVRQAPGQGLEWIGVINPESGD
TYYSEKFQGRVTLTADKSTSTAYMELSSLRSEDSAVYFCAKESLTGSSYDYWGQGTTLTV
SS
   900792-VH2 (SEQ ID NO: 33) 
QVQLVQSGAEVKKPGASVKVSCKASGYAFTNYLIEWVRQAPGQGLEWMGIINPESGD
TYYAQKFQGRVTMTADKSTSTAYMELSSLRSEDSAVYFCAKESLTGSSYDYWGQGTTLT
VSS
   900792-VH3 (SEQ ID NO: 34) 
QVQLQQSGAEVKKPGASVKVSCKASGYAFTNYLIEWVRQAPGQGLEWMGVINPESG
DTYYSQKFQGRVTMTADKSTSTAYMELSSLRSEDSAVYFCAKESLTGSSYDYWGQGTTL
TVSS
   900792-VH4 (SEQ ID NO: 35) 
QVQLVQSGAEVKKPGASVKVSCKASGYAFTNYLIEWVRQAPGQGLEWMGIINPESGD
TYYAQKFQGRVTLTADKSTSTAYMELSSLRSEDSAVYYCAKESLTGSSYDYWGQGTTLT
VSS
900792 light chain humanized sequences:
   900792-VL1 (SEQ ID NO: 37) 
DIQMTQSPSSLSASVGDRVTITCRASENIYSNLVWYQQKPGKAPQLLVYGARNLADG
VPSRFSGSGSGTQFSLTISSLQPEDFATYYCQHFYETPLTFGTGTKLEIK
   900792-VL2 (SEQ ID NO: 38) 
DIQMTQSPSSLSASVGDRVTITCRASENIYSNLVWYQQKPGKAPQLLVYGARNLQSGV
PSRFSGSGSGTQFSLTISSLQPEDFATYYCQHFYETPLTFGTGTKLEIK
   900792-VL3 (SEQ ID NO: 39)

DIQMTQSPSSLSASVGDRVTITCRASENIYSNLVWYQQKPGKAPQLLIYGARNLQSGV

PSRFSGSGSGTQFTLTISSLQPEDFATYYCQHFYETPLTFGTGTKLEIK

Note: The italic underlined sequences are antibody CDR sequences, and the CDR sequence division method is based on the IMGT format.

The above 4 900792 heavy chain humanized sequences and 3 900792 light chain humanized sequences were randomly combined to obtain 12 900792 humanized antibodies, which were expressed and purified by CHO-S cells respectively. By flow cytometry, Biacore, and other assay methods, the ability to bind to TSLP antigens, the activity of blocking TSLP from binding to receptors thereof, the non-specific binding characteristics, the thermal stability, and other indicators of the 900792 humanized proteins were screened, and multiple humanized anti-TSLP antigen-binding proteins having excellent properties were obtained.

### Example 3 Test of anti-TSLP humanized antibodies

### 3.1 Test of activity of 900792 humanized proteins binding to human TSLP-expressing cells

The activity of the 900792 humanized proteins binding to human TSLP-expressing cells (Baf3-hu-TSLP-3G11, Huabo Biopharm) was tested.

All the antigen-binding proteins were diluted to 30 µg/ml with PBS solution containing 1% BSA (1% BSA/PBS), then diluted 3-fold in 10 gradients for a total of 11 concentrations, and added at a density of 20 µL per well to a 96-well U-shaped plate. A negative control (1% BSA/PBS added only) was set up simultaneously. A suspension of the human TSLP-expressing cells (Baf3-hu-TSLP-3G11, Huabo Biopharm) at the logarithmic growth phase was centrifuged (300 g×5 min), and the medium was discarded. The cells were resuspended with 1% BSA/PBS to a live cell density of 1×10⁶/mL, added at a density of 20 µL (2×104 cells) per well to the 96-well U-shaped plate containing the anti-TSLP antigen-binding proteins, and incubated at room temperature for 30 min. Following incubation, the 96-well U-shaped plate was centrifuged (300 g×3 min) and the supernatant was discard. Then 100 ul of 1% BSA/PBS per well was added to the 96-well U-shaped plate to resuspend the cells. The cells were centrifuged (300 g×3 min) and the supernatant was discarded. The cells were washed twice. Then 20 µl of 1:200 diluted PE-goat anti-human-Fc (Jackson Immuno Research, #109-115-098) per well was added, and incubated at room temperature in the dark for 15 min. Following incubation, the 96-well U-shaped plate was centrifuged (300 g×3 min) and the supernatant was discarded. Then 100 µl of 1% BSA/PBS per well was added to the 96-well U-shaped plate to resuspend the cells. The cells were centrifuged (300 g×3 min) and the supernatant was discarded. The cells were washed three times and finally resuspended with 100 µL of 1% BSA/PBS per well. The fluorescence intensity of a PE channel was tested by a flow cytometer (BD, #Canto II).

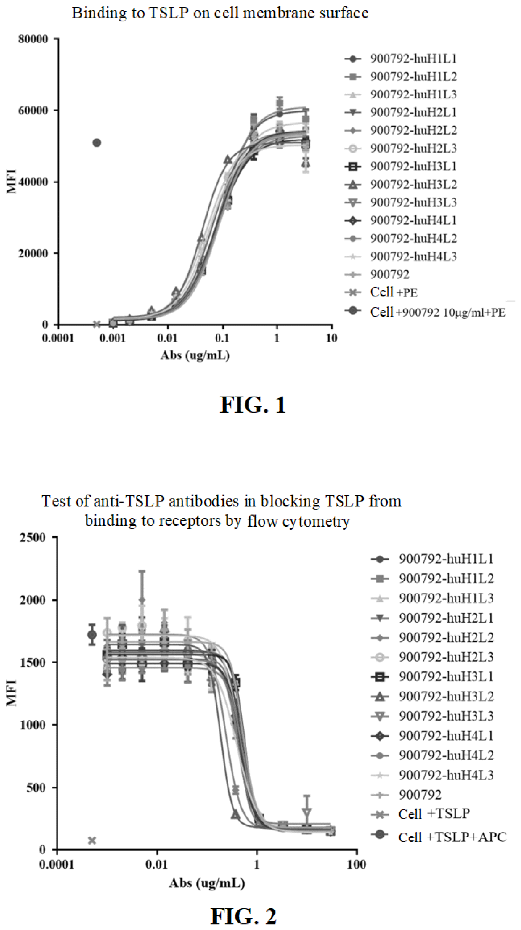

The experimental results are shown in FIG. 1:
Based on the curves of the activity and EC50 values of the 900792 and related humanized proteins in binding to the TSLP on the cell membrane surface, it was indicated that the 900792 humanized proteins have equivalent activity of binding to the human TSLP-expressing cells (Baf3-hu-TSLP-3G11, Huabo Biopharm).

### 3.2 Experiment on 900792 humanized proteins blocking TSLP from binding to TSLPR receptors on cell surface

The activity of the 900792 humanized proteins in blocking TSLP cytokines from binding to human TSLP receptor and IL7Ra expressing cells (Baf3-huTSLPR-IL7Ra-3H9-2, Huabo Biopharm) was tested.

Biotinylated Human TSLP (Acro, #TSP-H82EB-200UG) was diluted to 1.6 µg/ml with PBS solution containing 1% BSA. All antigen-binding proteins were diluted to 30 µg/ml and then diluted 2-fold in 10 gradients for a total of 11 concentrations. 10 µl of Biotinylated Human TSLP and 10 µl of antigen-binding proteins were mixed and added to a 96-well U-shaped plate (1% BSA and Biotinylated Human TSLP were mixed as negative control wells and positive control wells). Baf3-huTSLPR-IL7Ra-3H9-2 cells were diluted to 1×10⁶ cells/ml and added at a density of 20 µl per well to the 96-well U-shaped plate. Following mixing and incubation at room temperature for 30 min, the cells were centrifuged (300 g×3 min) and the supernatant was discarded. Then 100 µl of 1% BSA/PBS per well was added to the 96-well U-plate to resuspend the cells. The cells were centrifuged (300 g×3 min) and the supernatant was discarded. The cells were washed once. 20 µl of 1:200 diluted anti-Biotin antibodies APC-SA (BD, #554067) per well were added and incubated at room temperature for 30 min (without addition to the negative control wells). Then the cells were centrifuged (300 g×3 min) and the supernatant was discarded. 100 µl of 1% BSA/PBS per well was added to the 96-well U-plate to resuspend the cells. The cells were centrifuged (300 g×3 min) and the supernatant was discarded. The cells were washed twice, and finally resuspended with 100 µL of 1% BSA/PBS per well. The fluorescence intensity of an APC channel was tested by a flow cytometer (BD, #Canto II).

The experimental results are shown in FIG. 2:
Based on the curves and related IC50 values of the 900792 and related humanized proteins in blocking the TSLP from binding to the receptors on the cell surface, it was indicated that the 900792 and related humanized proteins have equivalent effects in blocking human TSLP from binding to cells expressing human TSLP receptors (Baf3-huTSLPR-IL7Ra-3H9-2, Huabo Biopharm), wherein 900792-huH2L2, 900792-huH3L2, and 900792-huH4L3 have better blocking effects.

### 3.3 Test of inhibitory effects of 900792 humanized proteins on functional activity of human TSLP

The inhibitory effects of the 900792 humanized proteins on the functional activity of human TSLP cytokines binding to cells expressing human TSLP receptors and IL7Ra coreceptors (Baf3-huTSLPR-IL7Ra-3H9-2, Huabo Biopharm) was tested.

Human TSLP (Acro, #TSP-H52Ha) was prepared to 3 ng/ml and added at a density of 50 µl per well to a 96-well black plate (only cell medium was added to negative control wells: RPMI160+10% FBS+10 mM HEPES mixed with the following cells). At the same time, all antigen-binding proteins were diluted to 50 µg/ml with medium, then 8 gradients were diluted at 8 times per gradient for a total of 9 concentrations, added at a density of 50 µl per well to the 96-well black plate containing the human TSLP, and mixed. Cells (Baf3-huTSLPR-IL7Ra-3H9-2, Huabo Biopharm) were resuspended to 2×10⁵ cells/ml and added at a density of 50 µl per well to the black plate (separate control wells in which only the cells and the human TSLP were mixed and incubated were set up). The cells were mixed and incubated in a 37°C incubator containing 5% CO₂ for 40-48 h. The cells were centrifuged (300 g×3 min) at the end of the culture, and 50 µl of the supernatant was discarded. 100 µl of CTG (CellTiter-Glo^{®} Luminescent Cell Viability Assay, Promega, #G7573) was added, and the cells were wrapped in tin foil, vibrated for 10 min, and then tested at a full wavelength.

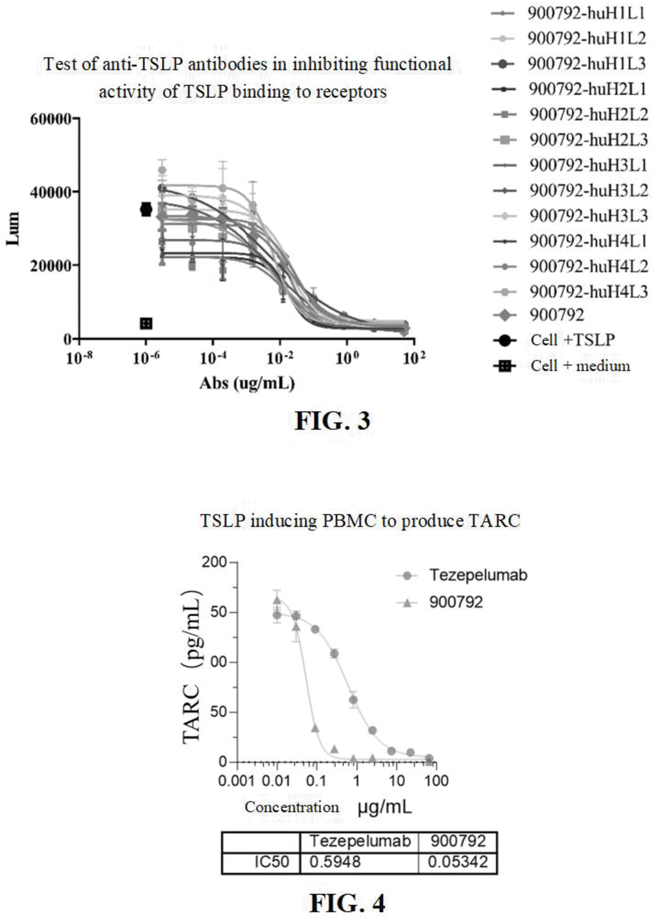

The experimental results are shown in FIG. 3:
Based on the curves and the relevant IC50 values of the 900792 and related humanized proteins in inhibiting the functional activity of the TSLP binding to the receptors on the cell surface, it was indicated that the 900792 and related humanized proteins can inhibit the functional activity of the human TSLP binding to the cells expressing the human TSLP receptor and the human IL7Ra coreceptor (Baf3-huTSLPR-IL7Ra-3H9-2, Huabo Biopharm), wherein 900792-huH1L3, 900792-huH2L3, 900792-huH3L2, and 900792-huH4L3 showed better inhibitory effects.

### 3.4 Test of affinity of 900792 humanized proteins binding to human and monkey TSLP proteins

A CM5 chip with fixed Anti-His antibodies was used to capture human TSLP antigens (ACRO, Cat#: TSP-H52Ha, Lot#: 2685a-82QF1-H2) and monkey TSLP antigen (R127A, R130S) Proteins, and His Tag (ACRO, Cat#: TSP-C52H4, Lot#: 3385a-9CHF1-QH) was used as a ligand. The antibody samples were diluted in gradient as analytes for a multi-cycle kinetic test. Under the conditions of Flow Rate: 30 µl/min, Association: 120 s, Dissociation: 600 s, 1:1 Binding Model, 1:1 Binding Model-Fit local, kinetic constants were analyzed, including the association rate constant (ka), dissociation rate constant (kd), and dissociation equilibrium rate constant (KD).

The experimental results are shown in Table 3 and Table 4.

All the 900792 humanized proteins bound to the human and monkey TSLP proteins, and the affinity of the most 900792 humanized proteins binding to the human and monkey TSLP proteins was not significantly different from that of the 900792 mouse chimeric proteins.

The 900792 chimera has a heavy chain sequence as set forth in SEQ ID NO: 40 and a light chain sequence as set forth in SEQ ID NO: 41. The sequence of 900792-huH1 is as set forth in SEQ ID NO: 42. The sequence of 900792-huH2 is as set forth in SEQ ID NO: 43. The sequence of 900792-huH3 is as set forth in SEQ ID NO: 44. The sequence of 900792-huH4 is as set forth in SEQ ID NO: 45. The sequence of 900792-huL1 is as set forth in SEQ ID NO: 46. The sequence of 900792-huL2 is as set forth in SEQ ID NO: 47. The sequence of 900792-huL3 is as set forth in SEQ ID NO: 48.

**Table 3 Test of affinity of 900792 and humanized proteins binding to human TSLP proteins**

| Analyte | Ligand | ka (1/Ms) | kd (1/s) | KD (M) |
|---|---|---|---|---|
| 900792 chimera | Human TSLP | 7.03E+05 | 1.57E-04 | 2.23E-10 |
| 900792-huH1L1 | Human TSLP | 7.48E+05 | 9.45E-05 | 1.26E-10 |
| 900792-huH1L2 | Human TSLP | 7.13E+05 | 1.26E-04 | 1.76E-10 |
| 900792-huH1L3 | Human TSLP | 7.28E+05 | 7.02E-05 | 9.64E-11 |
| 900792-huH2L1 | Human TSLP | 7.82E+05 | 1.10E-04 | 1.41E-10 |
| 900792-huH2L2 | Human TSLP | 7.33E+05 | 1.26E-04 | 1.72E-10 |
| 900792-huH2L3 | Human TSLP | 7.33E+05 | 1.26E-04 | 1.72E-10 |
| 900792-huH3L1 | Human TSLP | 7.37E+05 | 1.29E-04 | 1.75E-10 |
| 900792-huH3L2 | Human TSLP | 8.22E+05 | 1.08E-04 | 1.31E-10 |
| 900792-huH3L3 | Human TSLP | 7.55E+05 | 1.43E-04 | 1.89E-10 |
| 900792-huH4L1 | Human TSLP | 6.94E+05 | 8.15E-05 | 1.17E-10 |
| 900792-huH4L2 | Human TSLP | 7.43E+05 | 1.74E-04 | 2.34E-10 |
| 900792-huH4L3 | Human TSLP | 7.71E+05 | 1.21E-04 | 1.57E-10 |

**Table 4 Test of affinity of 900792 and humanized proteins binding to monkey TSLP proteins**

| Analyte | Ligand | ka (1/Ms) | kd (1/s) | KD (M) |
|---|---|---|---|---|
| 900792 chimera | Monkey TSLP | 3.47E+06 | 1.59E-02 | 4.58E-09 |
| 900792-huH1L1 | Monkey TSLP | 4.16E+05 | 3.39E-03 | 8.16E-09 |
| 900792-huH1L2 | Monkey TSLP | 3.33E+05 | 2.74E-03 | 8.23E-09 |
| 900792-huH1L3 | Monkey TSLP | 4.02E+05 | 3.21E-03 | 7.99E-09 |
| 900792-huH2L1 | Monkey TSLP | 3.19E+06 | 1.22E-02 | 3.82E-09 |
| 900792-huH2L2 | Monkey TSLP | 3.27E+06 | 1.65E-02 | 5.06E-09 |
| 900792-huH2L3 | Monkey TSLP | 3.22E+06 | 1.89E-02 | 5.88E-09 |
| 900792-huH3L1 | Monkey TSLP | 5.82E+06 | 3.62E-02 | 6.22E-09 |
| 900792-huH3L2 | Monkey TSLP | 6.47E+06 | 3.27E-02 | 5.05E-09 |
| 900792-huH3L3 | Monkey TSLP | 1.45E+07 | 8.70E-02 | 6.01E-09 |
| 900792-huH4L1 | Monkey TSLP | 2.09E+06 | 8.73E-03 | 4.17E-09 |
| 900792-huH4L2 | Monkey TSLP | 3.27E+06 | 1.30E-02 | 3.99E-09 |
| 900792-huH4L3 | Monkey TSLP | 2.37E+06 | 9.89E-03 | 4.18E-09 |

### 3.5 Test of non-specific adsorption effects of 900792 humanized proteins

The non-specific adsorption effects of the antigen-binding proteins on non-target molecules were determined by an SPR method.

By the quality control results of Lysozyme solution from chicken egg and Trypsin inhibitor type 1-S from Glycine max through Polyclonal rabbit anti-lysozyme and Anti-trypsin inhibitor antibody respectively, it could be determined that the activity of the Lysozyme solution from chicken egg and Trypsin inhibitor type 1-S from Glycine max was normal after amino coupling and fixation to a CM5 chip, that is, the activity of the fixed ligand remained good during the whole experiment. The PI of the Trypsin inhibitor type 1-S from Glycine max is 4.5, and the PI of the Lysozyme is 11.3. In an HBS-EP buffer system at pH 7.4, the Trypsin inhibitor type 1-S from Glycine max is highly negatively charged, and the Lysozyme is highly positively charged.

The experimental results are shown in Table 5.

In a case that the binding response values of all tested samples with the Lysozyme and the Trypsin are 20 RU or less, it can be considered that the currently tested samples have no obvious non-specific electrostatic binding effects.

**Table 5 Comparison of non-specific binding capacities of samples**

| Name | Lysozyme (RU) | Trypsin inhibitor (RU) | Inactivated carboxymethyl dextran (RU) | Carboxymet hyl dextran (RU) |
|---|---|---|---|---|
| Buffer | 0 | 0 | 0 | 0 |
| Polyclonal rabbit anti-lysozyme | 4935.6 | 1.6 | 0.3 | 4.1 |
| Anti-trypsin inhibitor | 10.5 | 4709.4 | 5.5 | 5.5 |
| 900792-huH1L1 | 1.8 | 5.7 | -0.8 | -0.7 |
| 900792-huH1L2 | 2.0 | 6.0 | -0.5 | -1.1 |
| 900792-huH1L3 | 1.0 | 5.9 | -0.5 | 0.1 |
| 900792-huH2L1 | 0.8 | 6.9 | -1.5 | -0.8 |
| 900792-huH2L2 | 1.4 | 6.8 | -0.8 | 0.6 |
| 900792-huH2L3 | 5.9 | 6.8 | -0.8 | -0.1 |
| 900792-huH3L1 | 0.9 | 5.6 | -0.6 | -1.0 |
| 900792-huH3L2 | 1.5 | 8.1 | -0.4 | 0.2 |
| 900792-huH3L3 | 1.9 | 8.7 | -0.8 | 0.6 |
| 900792-huH4L1 | 4.0 | 9.6 | -0.1 | 0.2 |
| 900792-huH4L2 | 3.3 | 8.6 | -0.6 | 0.0 |
| 900792-huH4L3 | 2.4 | 6.6 | -0.2 | 0.0 |
| 900792 chimera | 2.1 | 7.2 | -0.3 | 2.9 |

### 3.6 Test of thermal stability of 900792 humanized proteins

The steps for testing the thermal stability of the anti-TSLP antigen-binding proteins were as follows:
The melting temperature (Tm) and aggregation temperature (Tagg) of the anti-TSLP antigen-binding proteins were tested using a protein stability analyzer (Uncle, UNCHAINED LABS, US), wherein the temperature rise range of the Tm and Tagg was 25°C to 95°C, with a heating rate of 0.3°C.

The experimental results are shown in Table 6:
The results of the thermal stability test of the 900792 humanized proteins showed that the anti-TSLP humanized proteins have good performance in the thermal stability data Tm and Tagg.

**Table 6 Test of thermal stability data Tm and Tagg values of anti-TSLP antigen-binding proteins**

| Antibody | Tm value (°C) | Tagg value (°C) |
|---|---|---|
| 900792-huH1L1 | 68.8 | 67.7 |
| 900792-huH1L2 | 67.5 | 64.2 |
| 900792-huH1L3 | 67.3 | 66.5 |
| 900792-huH2L1 | 68.3 | 66.7 |
| 900792-huH2L2 | 67.4 | 64.6 |
| 900792-huH2L3 | 67.6 | 66.4 |
| 900792-huH3L1 | 68 | 65.8 |
| 900792-huH3L2 | 68.5 | 66.5 |
| 900792-huH3L3 | 65.7 | 64.4 |
| 900792-huH4L1 | 71.2 | 70 |
| 900792-huH4L2 | 69.6 | 67.9 |
| 900792-huH4L3 | 68 | 67.1 |

### 3.7 Test of 900792 humanized proteins in blocking functional activity of TARC

TSLP (Thymic stromal lymphopoietin) can stimulate dendritic cells or monocytes to produce chemokines TARC (Thymus and activation-regulated chemokines), and further recruit type II helper T cells and cause a type II inflammatory response, while TARC is not produced in a resting state. In an in vitro culture system, dendritic cells and monocytes in PBMC are stimulated by TSLP to assay the content of TARC produced. When anti-TSLP antibodies are added to neutralize the added TSLP, the production of the TARC is blocked, showing a dose-dependent relationship with the concentration gradient anti-TSLP antibodies, and thereby the neutralizing activity of the antibodies is evaluated.

Test method: Anti-TSLP antibodies were diluted to 2 µg/mL, then diluted 2-fold for a total of 7 concentrations, and added at a density of 50 µl/well to a 96-well plate. Then TSLP was diluted to 10 ng/mL, added at a density of 50 µl/well to the 96-well plate, mixed and incubated for 30 min. The cells were revived and centrifuged at 500 g for 5 min, and the supernatant was removed. The cells were resuspended in complete medium, counted, adjusted to a density of 2×10⁶ cells/mL, and added at a density of 100 µl/well to a cell plate. The incubated antigen-antibody mixture was added to the cells at a density of 100 µl/well, cultured in a 37°C incubator containing 5% CO₂ for 48 h, and then centrifuged to obtain the supernatant. The content of TARC in the cell culture supernatant was tested by ELISA.

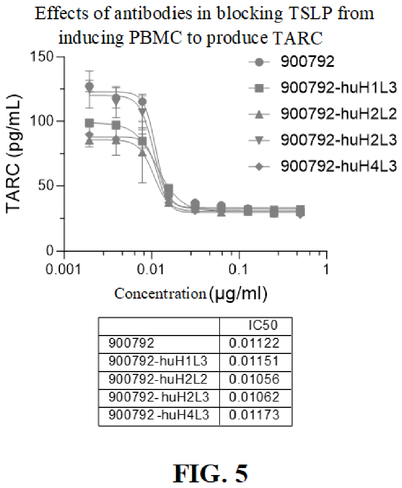

The experimental results are shown in FIG. 4, FIG. 5:
The 900792 humanized proteins are nearly 10 times better than a positive control drug Tezepelumab (sequence derived from patent US8163284) in blocking the functional activity of TARC.

All the 900792 humanized proteins can effectively block the functional activity of TARC, and have the similar activity to chimeric antibodies.

## Claims

1. An isolated antigen-binding protein, targeting TSLP, and comprising HCDR1, HCDR2, and HCDR3 of a variable region of the heavy chain VH, wherein the HCDR1 comprises an amino acid sequence as set forth in SEQ ID NO: 1, the HCDR2 comprises an amino acid sequence as set forth in SEQ ID NO: 2, and the HCDR3 comprises an amino acid sequence as set forth in SEQ ID NO: 3.

2. The isolated antigen-binding protein of claim 1, comprising H-FR1, wherein a C terminus of the H-FR1 is directly or indirectly connected to an N terminus of the HCDR1, and the H-FR1 comprises an amino acid sequence optionally selected from SEQ ID NO: 7, SEQ ID NO: 8, and SEQ ID NO: 9.

3. The isolated antigen-binding protein of any one of claims 1 to 2, comprising H-FR2, wherein the H-FR2 is located between the HCDR1 and the HCDR2, and the H-FR2 comprises an amino acid sequence optionally selected from SEQ ID NO: 10, SEQ ID NO: 11, SEQ ID NO: 12, and SEQ ID NO: 13.

4. The isolated antigen-binding protein of any one of claims 1 to 3, comprising H-FR3, wherein the H-FR3 is located between the HCDR2 and the HCDR3, and the H-FR3 comprises an amino acid sequence optionally selected from SEQ ID NO: 14, SEQ ID NO: 15, SEQ ID NO: 16, SEQ ID NO: 17, and SEQ ID NO: 18.

5. The isolated antigen-binding protein of any one of claims 1 to 4, comprising H-FR4, wherein an N terminus of the H-FR4 is connected to a C terminus of the HCDR3, and the H-FR4 comprises an amino acid sequence optionally selected from SEQ ID NO: 19 and SEQ ID NO: 20.

6. The isolated antigen-binding protein of any one of claims 1 to 5, comprising H-FR1, H-FR2, H-FR3 and H-FR4, wherein the H-FR1, H-FR2, H-FR3 and H-FR4 comprise a group of amino acid sequences selected from the group consisting of:
1) H-FR1: SEQ ID NO: 7, H-FR2: SEQ ID NO: 10, H-FR3: SEQ ID NO: 14, H-FR4: SEQ ID NO: 19;
2) H-FR1: SEQ ID NO: 8, H-FR2: SEQ ID NO: 11, H-FR3: SEQ ID NO: 15, H-FR4: SEQ ID NO: 20;
3) H-FR1: SEQ ID NO: 9, H-FR2: SEQ ID NO: 12, H-FR3: SEQ ID NO: 16, H-FR4: SEQ ID NO: 20;
4) H-FR1: SEQ ID NO: 8, H-FR2: SEQ ID NO: 13, H-FR3: SEQ ID NO: 17, H-FR4: SEQ ID NO: 20; and
5) H-FR1: SEQ ID NO: 9, H-FR2: SEQ ID NO: 12, H-FR3: SEQ ID NO: 18, H-FR4: SEQ ID NO: 20.

7. The isolated antigen-binding protein of any one of claims 1 to 6, comprising a variable region of the antibody heavy chain VH, wherein the VH comprises an amino acid sequence selected from SEQ ID NO: 31, SEQ ID NO: 32, SEQ ID NO: 33, SEQ ID NO: 34, and SEQ ID NO: 35.

8. The isolated antigen-binding protein of any one of claims 1 to 7, comprising LCDR1, LCDR2, and LCDR3 of a variable region of the light chain VL, wherein the LCDR1 comprises an amino acid sequence as set forth in SEQ ID NO: 4, the LCDR2 comprises an amino acid sequence as set forth in SEQ ID NO: 5 (GAR), and the LCDR3 comprises an amino acid sequence as set forth in SEQ ID NO: 6.

9. The isolated antigen-binding protein of any one of claims 1 to 8, comprising L-FR1, wherein a C terminus of the L-FR1 is directly or indirectly connected to an N terminus of the LCDR1, and the L-FR1 comprises an amino acid sequence optionally selected from SEQ ID NO: 21 and SEQ ID NO: 22.

10. The isolated antigen-binding protein of any one of claims 1 to 9, comprising L-FR2, wherein the L-FR2 is located between the LCDR1 and the LCDR2, and the L-FR2 comprises an amino acid sequence optionally selected from SEQ ID NO: 23, SEQ ID NO: 24, and SEQ ID NO: 25.

11. The isolated antigen-binding protein of any one of claims 1 to 10, comprising L-FR3, wherein the L-FR3 is located between the LCDR2 and the LCDR3, and the L-FR3 comprises an amino acid sequence optionally selected from SEQ ID NO: 26, SEQ ID NO: 27, SEQ ID NO: 28, and SEQ ID NO: 29.

12. The isolated antigen-binding protein of any one of claims 1 to 11, comprising L-FR4, wherein an N terminus of the L-FR4 is connected to a C terminus of the LCDR3, and the L-FR4 comprises an amino acid sequence as set forth in SEQ ID NO: 30.

13. The isolated antigen-binding protein of any one of claims 1 to 12, comprising L-FR1, L-FR2, L-FR3 and L-FR4, wherein the L-FR1, L-FR2, L-FR3 and L-FR4 comprise a group of amino acid sequences selected from the group consisting of:
1) L-FR1: SEQ ID NO: 21, L-FR2: SEQ ID NO: 23, L-FR3: SEQ ID NO: 26, L-FR4: SEQ ID NO: 30;
2) L-FR1: SEQ ID NO: 22, L-FR2: SEQ ID NO: 24, L-FR3: SEQ ID NO: 27, L-FR4: SEQ ID NO: 30;
3) L-FR1: SEQ ID NO: 22, L-FR2: SEQ ID NO: 24, L-FR3: SEQ ID NO: 28, L-FR4: SEQ ID NO: 30; and
4) L-FR1: SEQ ID NO: 22, L-FR2: SEQ ID NO: 25, L-FR3: SEQ ID NO: 29, L-FR4: SEQ ID NO: 30.

14. The isolated antigen-binding protein of any one of claims 1 to 13, comprising a variable region of the antibody heavy chain VL, wherein the VL comprises an amino acid sequence optionally selected from SEQ ID NO: 36, SEQ ID NO: 37, SEQ ID NO: 38, and SEQ ID NO: 39.

15. The isolated antigen-binding protein of any one of claims 1 to 14, comprising any one group of VH and VL selected from the group consisting of:
1) VH: SEQ ID NO: 31, VL: SEQ ID NO: 36;
2) VH: SEQ ID NO: 32, VL: SEQ ID NO: 37;
3) VH: SEQ ID NO: 32, VL: SEQ ID NO: 38;
4) VH: SEQ ID NO: 32, VL: SEQ ID NO: 39;
5) VH: SEQ ID NO: 33, VL: SEQ ID NO: 37;
6) VH: SEQ ID NO: 33, VL: SEQ ID NO: 38;
7) VH: SEQ ID NO: 33, VL: SEQ ID NO: 39;
8) VH: SEQ ID NO: 34, VL: SEQ ID NO: 37;
9) VH: SEQ ID NO: 34, VL: SEQ ID NO: 38;
10) VH: SEQ ID NO: 34, VL: SEQ ID NO: 39;
11) VH: SEQ ID NO: 35, VL: SEQ ID NO: 37;
12) VH: SEQ ID NO: 35, VL: SEQ ID NO: 38; and
13) VH: SEQ ID NO: 35, VL: SEQ ID NO: 39.

16. The isolated antigen-binding protein of any one of claims 1 to 15, comprising a constant region of the antibody heavy chain.

17. The isolated antigen-binding protein of claim 16, wherein the constant region of the antibody heavy chain is derived from a human IgG heavy chain constant region.

18. The isolated antigen-binding protein of any one of claims 16 to 17, wherein the constant region of the antibody heavy chain is derived from a human IgG1 heavy chain constant region.

19. The isolated antigen-binding protein of any one of claims 1 to 18, comprising a constant region of the antibody light chain.

20. The isolated antigen-binding protein of claim 19, wherein the constant region of the antibody light chain is derived from a human Igκ constant region.

21. The isolated antigen-binding protein of any one of claims 1 to 20, comprising an antibody or an antigen-binding fragment thereof.

22. The isolated antigen-binding protein of claim 21, wherein the antigen-binding fragment comprises Fab, Fab', Fv fragment, F(ab')2, F(ab)2, scFv, di-scFv, and/or dAb.

23. The isolated antigen-binding protein of claim 21, wherein the antibody is selected from one or more of the group consisting of: monoclonal antibodies, chimeric antibodies, humanized antibodies, and fully human antibodies.

24. A chimeric antigen receptor, comprising a targeting moiety, wherein the targeting moiety comprises the isolated antigen-binding protein of any one of claims 1 to 23.

25. A polypeptide molecule, comprising the isolated antigen-binding protein of any one of claims 1 to 23 or the chimeric antigen receptor of claim 24.

26. An immunoconjugate, comprising the isolated antigen-binding protein of any one of claims 1 to 23.

27. One or more isolated nucleic acid molecules, encoding the isolated antigen-binding protein of any one of claims 1 to 23, the chimeric antigen receptor of claim 24, or the polypeptide molecule of claim 25.

28. A vector, comprising the nucleic acid molecule of claim 27.

29. A cell, comprising the isolated antigen-binding protein of any one of claims 1 to 23, the chimeric antigen receptor of claim 24, the polypeptide molecule of claim 25, the immunoconjugate of claim 26, the nucleic acid molecule of claim 27, or the vector of claim 28.

30. A pharmaceutical composition, comprising the isolated antigen-binding protein of any one of claims 1 to 23, the chimeric antigen receptor of claim 24, the polypeptide molecule of claim 25, the immunoconjugate of claim 26, the nucleic acid molecule of claim 27, the vector of claim 28, and/or the cell of claim 29, and optionally a pharmaceutically acceptable carrier.

31. A method for preparing the isolated antigen-binding protein of any one of claims 1 to 23, comprising: culturing the cell of claim 29 under a condition that allows expression of the isolated antigen-binding protein.

32. Use of the isolated antigen-binding protein of any one of claims 1 to 23, the chimeric antigen receptor of claim 24, the polypeptide molecule of claim 25, the immunoconjugate of claim 26, the nucleic acid molecule of claim 27, the vector of claim 28, the cell of claim 29, and/or the pharmaceutical composition of claim 30 in the manufacture of a medicament for preventing, alleviating, and/or treating diseases and/or disorders.

33. The use of claim 32, wherein the diseases and/or disorders comprise TSLP related diseases.

34. The use of claim 32 or claim 33, wherein the diseases and/or disorders comprise inflammatory diseases or tumors.

35. A method for detecting TSLP in a sample, comprising application of the isolated antigen-binding protein of any one of claims 1 to 23, the chimeric antigen receptor of claim 24, the polypeptide molecule of claim 25, the immunoconjugate of claim 26, the nucleic acid molecule of claim 27, the vector of claim 28, the cell of claim 29, and/or the pharmaceutical composition of claim 30.

36. A reagent or kit for detecting TSLP in a sample, comprising the isolated antigen-binding protein of any one of claims 1 to 23, the chimeric antigen receptor of claim 24, the polypeptide molecule of claim 25, the immunoconjugate of claim 26, the nucleic acid molecule of claim 27, the vector of claim 28, the cell of claim 29, and/or the pharmaceutical composition of claim 30.
